# EUROPÄISCHE PATENTSCHRIFT

(11) **EP 1 526 915 B1**
(45) Veröffentlichungstag und Bekanntmachung des Hinweises auf die Patenterteilung: **17.10.2007**
(21) Anmeldenummer: 03784109.5
(22) Anmeldetag: 29.07.2003
(51) Int. Cl.: B01J 13/02, B01J 13/22

(54) **FARBKODIERTE LAYER-BY-LAYER MIKROKAPSELN ALS KOMBINATORISCHE ANALYSEBIBLIOTHEKEN UND ALS SPEZIFISCHE OPTISCHE SENSOREN**
COLOR COATED LAYER-BY-LAYER MICROCAPSULES SERVING AS COMBINATORY ANALYSIS LIBRARIES AND AS SPECIFIC OPTICAL SENSORS
MICROCAPSULES COUCHE PAR COUCHE A CODES DE COULEURS SERVANT DE BIBLIOTHEQUES D'ANALYSE COMBINATOIRES ET DE CAPTEURS OPTIQUES SPECIFIQUES

(30) Priorität: 02.08.2002 DE 10236409; 02.04.2003 DE 10315846
(43) Veröffentlichungstag der Anmeldung: 04.05.2005
(73) Patentinhaber: Capsulution Nanoscience AG, 12489 Berlin (DE)
(72) Erfinder: DAEHNE, Lars, 12587 Berlin (DE); BAUDE, Barbara, 14548 Caputh (DE); VOIGT, Andreas, 12621 Berlin (DE)
(74) Vertreter: Leidescher, Thomas
(86) Internationale Anmeldenummer: PCT/EP2003/008376
(87) Internationale Veröffentlichungsnummer: WO 2004/014540

(56) Entgegenhaltungen:
- WO-A-00/32542
- WO-A-02/17888
- GB-A- 2 135 954
- DAI Z ET AL: "LAYER-BY-LAYER SELF-ASSEMBLY OF POLYELECTROLYTE AND LOW MOLECULAR WEIGHT SPECIES INTO CAPSULES" ADVANCED MATERIALS, VCH VERLAGSGESELLSCHAFT, WEINHEIM, DE, Bd. 13, Nr. 17, 3. September 2001 (2001-09-03), Seiten 1339-1342, XP001129372 ISSN: 0935-9648

## Beschreibung

Die vorliegende Erfindung betrifft kombinatorische Bibliotheken auf der Basis von hohlen oder gefüllten Polyelektrolytkapseln, die mittels der Layer by Layer Methode hergestellten werden. Die LbL Methode ermöglicht die Kontrolle der Anzahl, der Konzentration und des Abstandes zwischen den Farbstoffmolekülen auf der Nanometerskala, wodurch sich eine höhere kodierte Informationsmenge in der Wand (Hülle) ergibt, als es von Partikeln (Beads, volle Mikropartikel) bekannt ist, die in ihrem Volumen oder an ihrer Oberfläche farblich kodiert sind. Der zweite Teil der Erfindung befasst sich mit der Möglichkeit, Kapseln mit unterschiedlichen Makromolekülen zu füllen, jedoch die Kapseln weiterhin permeabel für kleine Moleküle zu halten. Derartige farbkodierte Kapseln können als kombinatorische Fangbehälter verwendet werden, welche eine erhebliche Menge von spezifischen Substanzen aus einem Reaktionsgemisch aufnehmen können. Nachfolgend können die unterschiedlichen Kapseln mit unterschiedlichen Substanzen in ihrem Inneren aufgrund ihrer spezifischen Fluoreszenzsignale aussortiert werden. Diese kombinatorischen Bibliotheken können in vielen Gebieten in der Medizin, der Biologie und der Chemie angewendet werden.

Die Miniaturisierung von Assays und Mikrotiterplatten ist im Hinblick auf eine weitere Vergrößerung der Assayskapazität begrenzt. Eine alternative Methode eröffnen die auf Beads basierenden Bibliotheken. Neue Entwicklungen in der Flußzytometrie (Flow Cytometrie; z.B. COPAS^{™} bead flow sorting) erlauben einen Durchsatz von bis zu 100.000 Partikel pro Stunde. Daher könnten die auf Beads basierenden Bibliotheken die führende Technologie in Screening-oder Sammeloperationen werden.^{1-5,7}

In Z. Dai et. al, Advanced Materials, Bd. 13, Nr. 17, Seiten 1339 - 1342 werden Untersuchungen zum Schichtwachstum von LbL-Kapseln beschreiben. Zum Nachweis dieses Wachstums werden hochmolekulare Polyelektrolyte und niedermolekularen Fluoreszenzfarbstoffe verwendet. Die Fluoreszenzfarbstoffe bilden jeweils separate selbständige Schichten, die innerhalb der Kapselhülle zwischen Polyelektrolytschichten angeordnet sind.

In WO 02/17888 wird ein Verfahren zum kontrollierten Schalten der Permeabilität von nach dem Layer-by-Layer Prinzip hergestellten Kapseln beschrieben. Zum Nachweis der Permeabilitätsveränderung werden unter anderem Kapseln verwendet, die eine mit Fluoreszein und eine weitere mit Rhodamin markierte Schicht aufweisen. Zwischen den fluoreszenzmarkierten Schichten ist eine Polyelektrolytschicht angeordnet. Mittels Försterresonanzenergietransfer (FRET) zwischen den fluoreszenzmarkierten Schichten kann das Quellen der Kapselhülle nachgewiesen werden, da sich beim Quellen die Dikke der zwischengelagerten Polyelektrolytschicht ändert und so der Abstand der beiden fluoreszenzmarkierten Schichten verändert wird.

Wir haben hohle farbkodierte Kapseln aus Polyelektrolyten⁶ hergestellt. Die farbkodierten Kapseln können wie Beads sortiert werden, jedoch sind sie hohl und können viele Bindungsstellen sowohl auf der Wandoberfläche als auch in ihrem Inneren aufweisen.

Diese Kapseln haben im Vergleich zu der Beads-Technologie verschiedene Vorteile:
1. Ihrer Masse ist sehr gering. Daher fallen Sie aus Lösungen mit unterschiedlicher Dichte wesentlich langsamer als Beads aus.
2. Infolge ihrer dünnen Wand und dem gleichen oder ähnlichem Material im Inneren wie im Äußeren ist die Lichtstreuung sehr klein. Bei Beads führen Unterschiede im Brechungsindex zwischen Bead und dem Lösungsmittel (gewöhnlich Wasser) zu starker Lichtstreuung, die den Sortierprozeß im Flußzytometer beeinträchtigt.
3. Reaktionen sind bei Beads nur an deren Oberfläche möglich. Daher ist die Anzahl ihrer Bindungsstellen stark begrenzt. Im Falle unserer Kapseln können die äußere Wandoberfläche, die innere Wandoberfläche und das gesamte Volumen der Kapseln für Reaktionen genutzt werden. Eine Kapseln (oder ein Bead) mit einem Durchmesser von 5 µm hat eine äußere Oberfläche von 78 µm² und ein Volumen von 65 µm³. Unter Annahme einer Konzentration der Bindungsstellen von 0.1 M weist ein Bead lediglich etwa 9 x 10⁴ Bindungsstellen auf, wohingegen eine Kapseln etwa 5000 mal mehr Bindungsstellen, nämlich 4 x 10⁸ Bindungsstellen aufweist.
4. Die Farbstoffmarkierungen können mit ausreichendem Abstand untereinander aufgebracht werden, um Wechselwirkungen wie die Bildung von H- oder J-Aggregaten, Selbstlöschung oder Förster-Resonanzenergietransfer zu vermeiden, welche die Fluoreszenzsignale im Falle der Markierung der Festkörperphase mit unterschiedlichen Farbstoffen stören. Dies gestattet mehr kombinatorische Möglichkeiten.
5. Der Innenraum der Kapseln kann mit hochaktiven Biowirkstoffen wie Enzyme, DNS oder dergleichen oder mit spezifisch funktionalisierten Polyelektrolyten gefüllt werden, welche ein selektives Fangen von Reaktionspartnern aus der Lösung durch Bioreaktionen, Physi- oder Chemisorption ermöglichen. Nachfolgend können die kodierten Kapseln aussortiert werden.
6. Die kodierte Information kann durch die Anzahl der Farbstoffen, ihr Verhältnis untereinander und durch abstandsabhängige Wechselwirkungen untereinander eingestellt werden. Bei den bekannten fluoreszierenden Beads⁴ sind solche Wechselwirkungen unerwünscht, da der Abstand zwischen den Farbstoffmolekülen nicht kontrollierbar ist.
7. Herstellung von hohlen kodierten Kapseln und Nutzung ihres Innenraums für die Immobilisierung von Makromolekülen (Polyelektrolyte, Proteine, Enzyme). Die funktionalisierten Makromoleküle können komplementäre Verbindungen aus Reaktionslösungen durch Physisorption, Chemisorption biologische Bindungen herausfischen.

Die vorliegende Erfindung betrifft Sensoren, die mittels der Layer-by-Layer LbL Methode auf Kolloiden mit Durchmessern kleiner als 100 µm aufgebaut werden und auf chemische Stoffe oder physikalische Meßgrößen ansprechen. Das kolloide Templat kann gegebenenfalls in einem Folgeschritt herausgelöst werden, so dass hohle Kapseln entstehen.

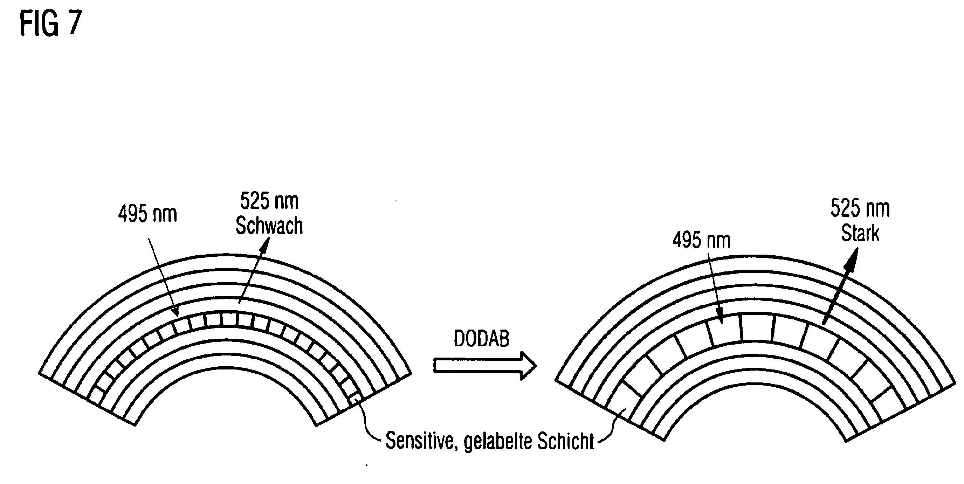

Die Sensorwirkung wird durch eine Schicht definierter Dicke eines speziellen Materials erreicht, dass bei Änderung der Konzentration eines Stoffes in der umgebenden Lösung oder bei der Veränderung physikalischer Parameter entweder quillt oder schrumpft. Zur Detektion dieses Prozesses wird die Emission von Fluoreszenzfarbstoffen verwendet. Eine Variante der Wirkungsweise ist in Abbildung 7 dargestellt:

Das sensitive Material wird kovalent mit einem Fluoreszenzfarbstoff in vergleichsweise hoher Konzentration (Masse Material: Masse Farbstoff < 500:1) verknüpft. Der Farbstoff zeichnet sich dadurch aus, dass er leicht mit sich selbst Dimere/Aggregate bildet. Wird das gelabelte Material in einer Kapselwand als mindestens eine homogene Schicht mit einer Dicke von 1 nm bis 1 µm eingebracht, führt ein Selfquenchingprozeß bei der Bildung von Dimeren oder H-aggregaten zu einer Löschung der Fluoreszenz des Farbstoffmonomeren, wohingegen bei Bildung von J-Aggregaten oder Excimeren eine neue Emissionsbande bei niedrigerer Energie entsteht. Bei Quellung/Schrumpfung der Schicht in der Kapselwand kann das Signal über die Intensität oder die Lebensdauer der Monomerfluoreszenz detektiert werden, bzw. über das Verhältnis von Monomerfluoreszenz zur Fluoreszenz des J-Aggregates bzw. Excimeren.

Im allgemeinen weisen die erfindungsgemäßen Kapseln, die bevorzugt einen Durchmesser kleiner als 100 µm haben, eine Hülle auf, die aus mindestens drei Polyelektrolytschichten aufgebaut ist, wobei eine der drei Polyelektrolytschichten mit einem Farbstoff markiert ist. Dieser Farbstoff, bei dem es sich um einen Fluoreszenzfarbstoff oder emittierende (fluoreszierende) Nanopartikel (Partikel mit einer Größe bevorzugt kleiner 1 nm) handeln kann, dient beispielsweise zur Identifizierung der Kapseln. In diesem Fall werden die Kapseln zur Markierung bzw. Codierung von technischen Erzeugnissen, Partikeln, Zellen, Gewebe, Organen oder Organismen biologischen Ursprungs verwendet, so daß anhand der Fluoreszenz des Farbstoffes deren Herkunft festgestellt und identifiziert werden kann. Andererseits können die Kapseln auch als Sensoren dienen, die auf veränderte Umgebungsbedingungen mit Änderung der Fluoreszenz des Farbstoffs meßbar reagieren. Schließlich können die Kapseln auch als 'Fangbehälter' verwendet werden, um Substanzen aus Lösungen zu entfernen bzw. zu identifizieren. Kapseln, die jeweils mit unterschiedlichen Farbstoffen markiert sind und spezifisch mit jeweils einer anderen Substanz reagieren, beispielsweise durch spezifische Bindungsstellen, eignen sich als Bibliothek von Reporter-Partikel zur Identifikation von Substanzen und/oder der Markierung von Prozessen. Es liegt im Rahmen der Erfindung, diese Anwendungen miteinander zu kombinieren.

Unter Polyelektrolyten werden im Rahmen der Erfindung insbesondere wasserlösliche Moleküle oder Aggregate verstanden, die mindestens 2 Ladungen, bevorzugt sogar mindestens drei Ladungen tragen. Bei einer Vielzahl von Polyelektrolyten liegen sogar deutlich mehr Ladungen vor. Zu den Polyelektrolyten zählen im Rahmen der Erfindung insbesondere organische Polyelektrolyte, Nanopartikel, Polyampholyte sowie Verbindungen und Komplexe aus organischen Polyelektrolyten und niedermolekularen Substanzen, z.B. Tenside.

Bei den Polyelektrolytschichten handelt es sich insbesondere um Schichten, die im wesentlichen etwa eine Monolage des entsprechenden Polyelektrolyts dick sind. Solche Polyelektrolytschichten lassen sich z.B. durch Layer-by-Layer Verfahren aufbringen. Bei diesen werden Polyelektrolyte abwechselnder Polarität aufgebracht, wobei sich Polyelektrolyte solange an bestehende Polyelektrolytschichten anlagern, bis die Ladungen der bereits bestehenden Schicht abgesättigt sind.

Mehrschichtige Polyektrolytkapseln, die auch aus unterschiedlichen Polyelektrolytschichten bestehen können, lassen sich beispielsweise nach dem Layer-by-Layer Verfahren herstellen, das in der DE 198 12 083 A1, DE 199 07 552 A1, EP 98 113 181, WO/47252 US 6,479,146 beschrieben ist. Die im weiteren im Zusammenhang mit FRET beschriebenen Beispiele stellen keine erfindungsgemäßen Beispiele dar, tragen jedoch für das Verständnis der Erfindung bei.

Sofern die Kapseln als Sensor dienen, können im Gegensatz zur Erfindung zwei der drei Hüllschichten mit jeweils einem unterschiedlichen Farbstoff markiert werden. Die nicht mit Fluoreszenzfarbstoffen markierte dritte Polyelektrolytschicht liegt dabei zwischen den beiden markierten Polyeleklrolytschichten. Dadurch weisen diese einen gewissen Abstand zueinander auf, der etwa der Dicke, beispielsweise 0.1 nm bis 10 nm, der nicht markierten mittleren dritten Schicht entspricht. Die Dicke der Polyelektrolytschicht hängt dabei unter anderem von dem verwendeten Polyelektrolyt ab. Ändert sich diese Dicke, z.B. als Reaktion auf veränderte Umgebungsbedingungen, so ändert sich die Stärke der Kopplung zwischen den Farbstoffmolekülen. Daher kann auch von einer sensitiven Schicht (sensorischen Zwischenschicht) gesprochen werden. Die Änderungen sind meßbar und dienen als Maß für die Veränderung der Schichtdicke. Bei den Umgebungsbedingungen, deren Änderung zu einer Änderung der Schichtdicke der nichtmarkierten Schicht führen, kann es sich um pH-Wert, Salzkonzentration, Temperatur, adsorbierte Komponenten, Enzyme, Konzentration eines Stoffes, physikalische Parameter, das Lösungsmittel beeinflussende oder mit der sensitiven Schicht reagierende Komponenten sowie mischbare Lösungsmittelanteile handeln. Insbesondere organische Polyelektrolyte reagieren empfindlich auf veränderte Umgebungsbedingungen. So führt beispielsweise eine Temperaturänderung zur Veränderung der Wasseraufnahmefähigkeit der organischen Polyelektrolyte und damit zu einer Schichtdickenänderung. Ein Beispiels ist hier PAH.

Sensorische Kapseln können erfindungsgemäß jedoch auch lediglich mit einem Farbstoff markiert sein. In diesem Fall ist der Farbstoff in hoher Konzentration an sensitives Material innerhalb einer Polyelektrolytschicht gebunden, wobei das sensitive Material auf die veränderten Umgebungsbedingungen durch Volumenzu- oder -abnahme reagieren kann. Die hohe Konzentration der Farbstoffe führt zu einem Selfquenching, beispielsweise durch Dimerbildung, oder zur Ausbildung neue Emissionsbanden im Falle der Bildung von Excimeren. Auch hier sind diese Prozesse stark von dem Abstand der Farbstoffe abhängig, so daß eine Änderung der Schichtdicke auch zu einem Änderung des Abstandes der Farbstoffe führt.

Sofern die Kapseln als 'Fangbehälter' dienen, weisen sie spezifische Bindungsstellen für die zu fangenden Moleküle auf. Die Bindungsstellen können sich im Inneren der Kapseln oder auf deren Hülle befinden. Kapseln mit unterschiedlichen Bindungsstellen können mit unterschiedlichen Farbstoffen markiert werden, so daß eine nachfolgende Sortierung anhand der Fluoreszenz möglich ist Dadurch können selektiv Substanzen, z.B. Proteine, aus Lösungen gewonnen werden.

### Beschreibung der Experimente

### Farbstoffmarkierung von Polyelektrolyten:

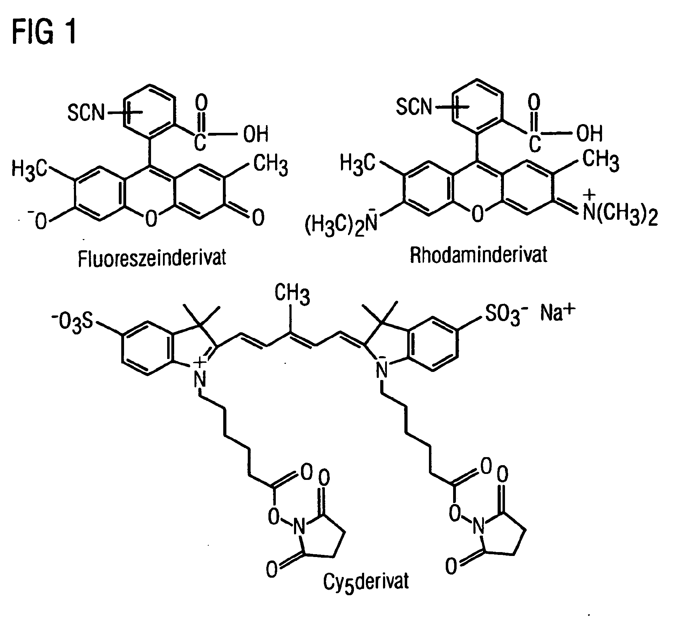

PAH wurde mit den Farbstoffderivaten Fluoreszeinisothiocyanat, Tetramethylrhodaminisothiocyanat und einem Derivat von CY5 markiert. Die Formeln sind in Abbildung 1 dargestellt. Die Markierungsreaktionen wurde entsprechend allgemeiner Herangehensweise bei der Markierung von Proteinen durchgeführt. Anstelle eines Hydrogenkarbonatpuffers wurde NaOH für die Aktivierung von ca. 30% der PAH Gruppen verwendet. Das Reaktionsgemisch wurde gegen Wasser dialysiert. Nach Zugabe von HCL zu der markierten PAH-Lösung zur Einstellung eines pH-Wertes von 4 - 5 wurde die Lösung lyophilisiert. Der Markierungsgehalt wurden mittels UV/Vis-Spektmskopie bestimmt und betrug für PAH-F1 53:1, PAH-Rho 580:1, PAH-Cy5 500:1 (Verhältnis der PAH Einheiten : Anzahl der markierten Moleküle). Die Ausbeute der Markierung betrug ca. 80% für Fluoreszein, 20% für Rhodamine und 40% für Cy5. Jedes PAH wurde nur mit einem Farbstoff markiert, da ein gleichzeitiges Markieren einer PAH-Kette den Nachteil der Selbstlöschung oder des Förster Resonanz Energie Transfer in sich birgt.

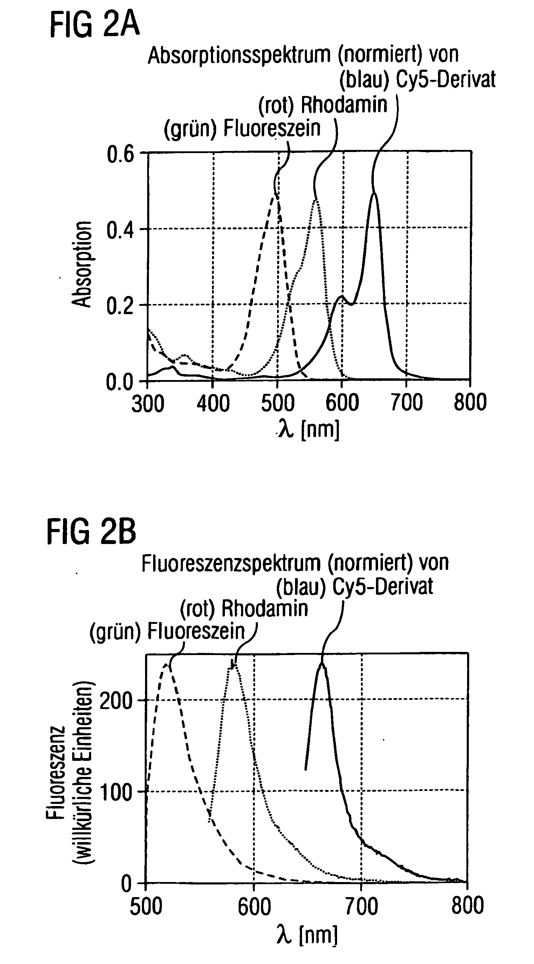

Die Absorptions- und Fluoreszenzspektren der Farbstoffe sind in Abb. 2a und b gezeigt. Die Absorptionsmaxima der drei markierten PAH-Polymere wurden zu 495, 557 und 648 nm bestimmt. Die Fluoreszenzmaxima betragen 520, 582 und 665 nm, wobei die Absorptionswellenlänge für die Anregung verwendet wurde.

### HersteUung der Kapseln:

3 µm große Silicatemplate wurden mit 10 alternierenden Schichten von Poly(allylaminhydrochlorid) (PAH, MW 60 000 g/mol) und Poly(styrolsulfonat) (PSS, MW 70 000 g/mol) beschichtet.⁹ Um unterscheidbare Wände zu erhalten, wurden unterschiedlich markierte PAH-Polymere für die Beschichtung verwendet. Für die Färbung der Kapseln wurde lediglich eine Schicht des jeweiligen PAH verwendet. Nur im Falle von Cy5 wurden aufgrund der geringeren Fluoreszenzquantenausbeute und des geringen Farbstoffgehalts 2 Schichten zur Markierung verwendet. Es wurde versucht, einen gewissen Abstand zwischen den unterschiedlichen Farbstoffschichten zur Vermeidung von Förster-Resonanzenergietransfer einzuhalten. Folgende Kapseln wurden hergestellt, wobei die Kapseln 2,5 bis 7 nicht-erfindungsgemäβe Vergleichskapseln sind:

**Tabelle 1: Farbkodierte Kapseln mit unterschiedlichen Arten von PAH-Farbstoff Schichten**

| Schicht/Kapsel | 1. | 2. | 3. | 4. | 5. | 6. | 7. | 8. |
|---|---|---|---|---|---|---|---|---|
| 1. PAH | - | - | - | - | - | - | - | - |
| 2. PSS | - | - | - | - | - | - | - | - |
| 3. PAH | - | - | - | Cy5 | Cy5 | Cy5 | Cy5 | - |
| 4. PSS | - | - | - | - | - | - | - | - |
| 5. PAH | Rho | Rho | Fluo | Cy5 | Cy5 | Cy5 | Cy5 | - |
| 6. PSS | - | - | - | - | - | - | - | - |
| 7. PAH | - | - | - | - | Fluo | Rho | Fluo | - |
| 8. PSS | - | - | - | - | - | - | - | - |
| 9.PAH | - | Fluo | - | - | - | - | Rho | - |
| 10. PSS | - | - | - | - | - | - | - | - |

Hohle Kapseln wurde durch Auflösen des Silicatemplats mittels Fluorwasserstoffsäure und Waschen mittels Wasser gewonnen.

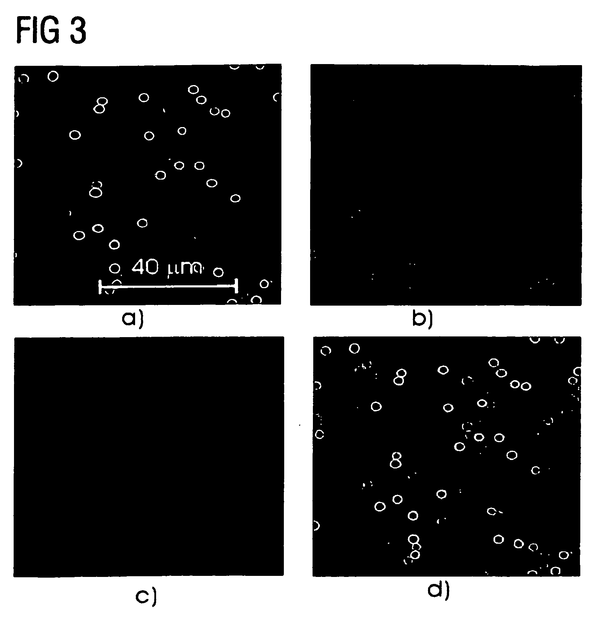

Die Kapseln wurden mittels konfokaler Laserscanningmikroskopie unter gleichzeitiger Verwendung von 3 unterschiedlichen Kanälen untersucht (Abb. 3a - c). Die Anregungswellenlänge der Laser war 488 nm für Fluoreszein, 543 nm für Rhodamin und 633 nm für Cy5. Die Detektoren wurden auf maximale Emission der Farbstoffe und auf einen minimalen Überlapp ihrer Fluoreszenzemission eingestellt. Die Laserintensitäten und die Detektorempfindlichkeiten wurden auf etwa gleiche Signalintensität für jeden Kanal eingestellt. Die Überlagerung der 3 Kanäle ergab 7 verschieden gefärbte Kapseln (Abb. 3d).

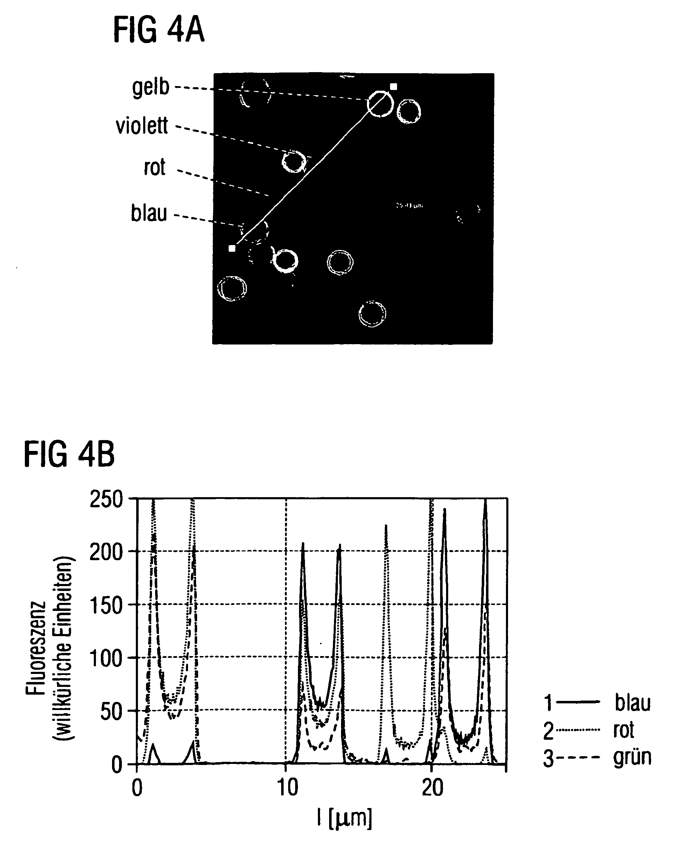

Eine quantitative und sichere Methode zur Unterscheidung der verschiedenen Kapseln bietet die Analyse der Fluoreszenzintensitäten entlang eines Profils durch die Kapseln. Die Profile zeigen die Verteilung der Fluoreszenzintensitäten von unterschiedlichen Kanälen der gleichen Kapsel. Abbildung 4a zeigt zum Beispiel das Profil der Kapseln 2, 7, 1 und 5.

Die Fluoreszenzintensitäten pro Farbstoffschicht sind unterschiedlich für unterschiedlich gefärbte Kapseln, was auf Resonanzenergieeffekte und unterschiedlichem Gehalt an adsorbiertem Material zurückgeführt werden kann. Der Resonanzenergietransfer kann bei Verwendung mehrerer Schichten zwischen den Farbstoffschichten deutlich reduziert werden. Oberhalb eines Abstands von 6 nm (ca. 4 Schichten) treten nahezu keine Wechselwirkungen zwischen den Farbstoffmolekülen mehr auf.

Für die Sensoranwendungen wurden die Kapseln 2 und 3 aus Tabelle 1 verwendet. Es wurde von uns gefunden, dass PAH/PSS Schichten bei Zugabe von Lösungen quarternärer Alkylammoniumsalze je nach Kettenlänge stark aufquellen oder auch schrumpfen. Ein starkes Quellen von (PAH/PSS)₅ Kapseln von 3 µm bis auf 5,7-6,0 µm wird bei Zugabe einer 0,05 M Dodecyltrimethylammomiumbromidlösung (DODAB) gefunden. Bei Verdoppelung des Kapseldurchmessers wird sich bei isotroper Quellung der Schichten der Abstand zwischen den Farbstoffschichten ebenfalls verdoppeln, wohingegen sich das Volumen einer Schicht um den Faktor 8 vergrößert.

Im nicht-enfindungsgemäßen Vergleichsexperiment 1 wurde die Kapsel 2 verwendet. Die Konzentration an Rhodamin und Fluoreszein in der Kapselwand wurde UV/NIS spektroskopisch vor und nach dem Quellungsvorgang bestimmt. Der mittlere Abstand zwischen beiden Farbstoffschichten betrug vor der Behandlung etwa 4,5 nm und danach nahezu 9 nm. Die Änderung des FRET Signals (λ_{exc} = 495 nm, λₑₘ = 578 nm) wurde mit dem Fluoreszenzspektrometer während des Quellvorgangs verfolgt. Durch die Quellung der Schichten verringerte sich die FRET Signalintensität bei Reaktion mit 0,05 M DODAB um 86%.

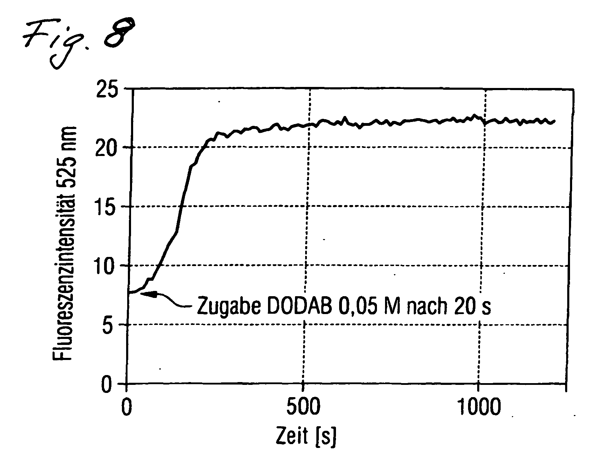

Im Experiment 2 gemäß einem Ausführungsbeispiel des Erfindung wurde die Kapselsorte 3 verwendet. Durch die hohe Fluoreszeinkonzentration in der einen PAH Schicht tritt ein effizienter Quenchprozeß auf. Nach Zugabe von 0,05 M DODAB Lösung vergrößert sich das Volumen der PAH Schicht etwa um den Faktor 8. Durch die verringerte Selbstlöschung des Farbstoffes erhöht sich dadurch die Fluoreszenz der Kapseln um 290 % (Abbildung 8).

### Füllen der Kapseln mit reaktiven Makromolekülen:

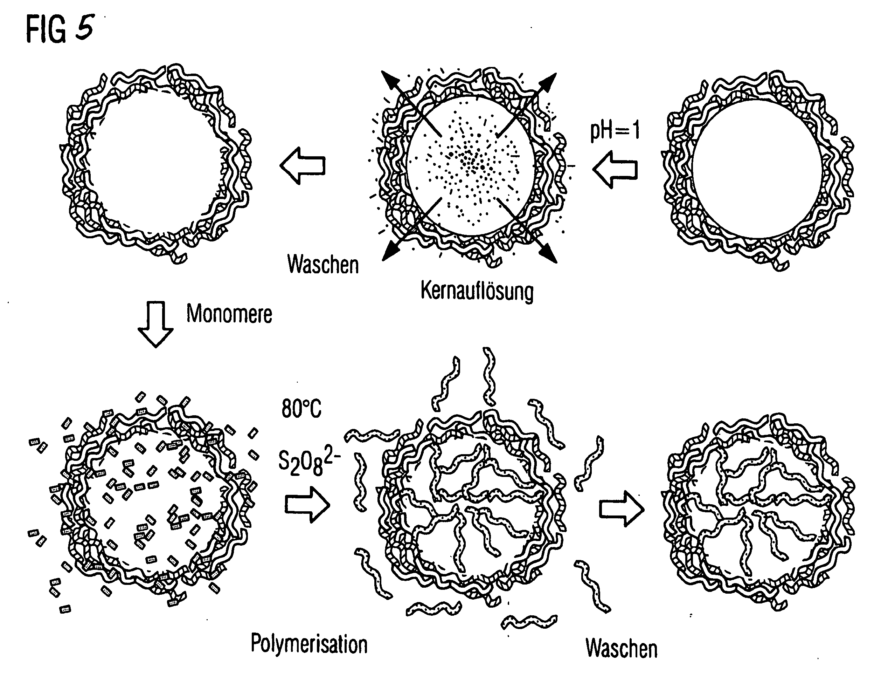

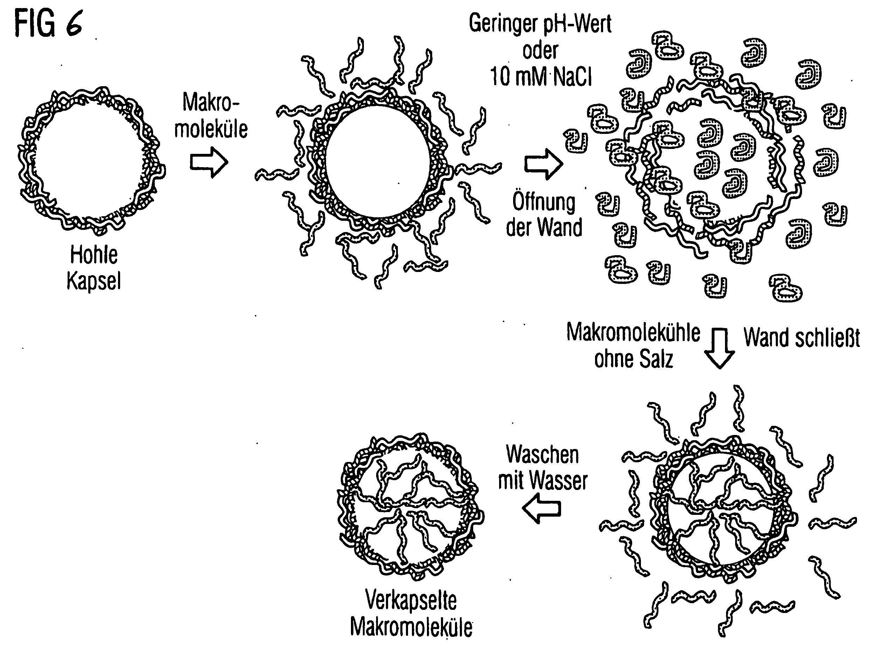

Es gibt drei unterschiedliche Wege für die Immobilisierung von Makromolekülen im Inneren der Kapseln:
1. "Schiff in Flasche" Synthese (ship in bottle synthesis) von Polymeren innerhalb der Kapseln (Abbildung 5).¹²
2. Schalten der Permeabilität von spezifischen Kapseln für korrespondierende Makromoleküle mittels Salze oder pH-Änderungen (Abbildung 6).¹¹
3. Bilden eines Niederschlags eines instabilen Komplexen aus den Makromolekülen und einer Hilfssubstanz auf dem kolloiden Templat. Nachfolgendes Einkapseln des Materials durch die übliche LbL-Methode und Aufflösen des Kerns und des Makromolekülkomplexes.⁸

Weitere vorteilhafte Ausgestaltungen der erfindungsgemäßen Kapseln sowie deren Verwendung sind nachfolgend angeführt, wobei sämtliche Ausgestaltungen beliebig miteinander kombinierbar sind:
- Kapseln aus Polyelektrolyt-Multischichten hergestellt nach dem Layer-by-Layer Verfahren und kleiner als 100 µm für Kodierungs- und sensorische/diagnostische/analytische Anwendungen und enthaltend
   a) eine definierte Zuordnung von farbstoffmarkierten Polyelektrolyten zur Schichtzahl,
   b) eine definierte Zuordnung von farbstofffreien Polyelektrolyten zur Schichtzahl,
   c) eine definierte Zuordnung von sensorischen Polyelektrolyten oder sensorisch reaktiven Beschichtungskomponenten zur Schichtzahl
   d) eine definierte Zuordnung von Wechselwirkungen der Markierungen unterschiedlicher Schichten
- Kapseln mit Kern oder ohne Kern als Hülle enthaltend das Lösungsmittel oder eine Lösung anderer Zusammensetzung.
- Kapseln mit einer Schicht mit einem Fluoreszenzfarbstoff in einer Dichte, die zur Selbstwechselwirkung (Selbstlöschung) innerhalb der Schicht führen kann und durch Änderungen von Komponenten oder Bedingungen des Mediums oder der Umgebung messbar beeinflusst werden kann und dafür als Sensor dienen kann.
- Kapseln, wobei die Kapseln kleiner als 10µm, bevorzugt kleiner als 1 µm sind.
- Kapseln mit einem modifizierten Kern, der sensorische Funktionen oder Kodierungseigenschaften besitzen kann.
- Verwendung der Kapseln als Bibliothek von Reporter-Partikeln oder kodierten Farbpartikeln zur Identifikation von Substanzen und/ oder der Markierung von Prozessen.
- Verwendung der Kapseln in der medizinischen Diagnostik, der kombinatorischen Chemie, der Genomik und Proteomik, der Biologie und Biotechnologie und der Technik.
- Verwendung der Kapseln zur Codierung von technischen Erzeugnissen.
- Verwendung der Kapseln zur Markierung von Partikel, Zellen, Geweben, Organen und Organismen biologischen Ursprungs.
- Zusammensetzung für die Identifizierung von Substanzen, wobei die Zusammensetzung mindestens zwei Arten von Kapseln mit einem Durchmesser weniger als 100 µm aufweist, wobei die Kapseln einen Kern und eine Hülle besitzen und die Hülle mindestens drei Schichten aufweist, wobei eine dieser Schichten mit einem Farbstoff markiert ist.
- Zusammensetzung aufweisend zumindest 3 Arten von Kapseln.
- Zusammensetzung, wobei die Kapseln einen mittleren Durchmesser von weniger als 10 µm, bevorzugt weniger als 1 µm besitzen.
- Zusammensetzung nach einem der vorangegangenen Ansprüchen, wobei die Hüllen aus Polyelektrolytschichten bestehen.

Figuren 1 bis 8 zeigen verschiedene Ausführungsformen der Erfindung.

Figur 1 zeigt die Struktur der verwendeten Fluoreszenzfarbstoffe.

Figur 2a) zeigt das Absorptionsspektrum (normalisierte Intensität) und Figur 2b) das Fluoreszenzspektrum (normalisierte Intensität) von PAH-F1, PAH-Rho und PAH-Cy5, normalisierte Intensität.

In Figur 3 sind konfokale Bilder einer Mischung von farbkodierten Kapseln dargestellt. 3 a) zeigt den Fluoreszeinkanal, d.h. die Fluoreszenz von Fluoreszein, 3 b) den Rhodaminkanal, 3 c) den Cy5 Kanal und 3 d) die Überlagerung der drei Farbkanäle.

Figur 4 zeigt einer Mischung der gefärbten Kapseln 2, 7, 1, 5. Für die Aufnahmen wurde ein konfokales Fluoreszenzmikroskop verwendet. In Fig. 4 a) ist das Überlagerungsbild der drei Farbkanäle des Fluoreszenzmikroskops und in 4 b) das Profil der Fluoreszenzintensität entlang der weißen Linie in Fig. 4 a) zu sehen.

In Figur 5 sind die prinzipiellen Schritte der sogenannten "Schiff in Flasche" Synthese von Polymeren innerhalb der Kapseln dargestellt. Nach der Auflösung des Kerns, der als Templat zur Beschichtung mit den Polyelektrolyten gedient hat, passieren Monomere die Hülle und gelangen in das Innere der Kapsel. Bei geeignet gewählten Bedingungen polymerisieren die Monomere und können daher die Hülle nicht mehr passieren. In einem abschließenden Waschschritt werden die Polymere außerhalb der Kapseln aus der Lösung entfernt. Zurück bleiben eingekapselte Monomere.

Figur 6 zeigt das Prinzip des Beladens von MF Kapseln (8 Schichten) durch Schalten der Permeabilität von speziellen Kapseln für korrespondierende Makromoleküle durch Salz oder pH-Wert. Durch Änderung des Salzgehaltes und/oder des pH-Wertes können die Poren der Hüllen vergrößert und damit die Permeabilität erhöht werden. Dies erlaubt auch größeren Markromolekülen das Eindringen in die Kapseln. Abschließend wird der pH-Wert und/oder der Salzgehalt wieder auf die Anfangswerte zurückgeführt; die Poren verschließen sich wieder bzw. werden kleiner. Die in die Kapseln eingedrungenden Makromoleküle können die Hülle nun nicht mehr passieren.

Figur 7 zeigt den schematischen Aufbau und die Wirkungsweise der oben beschriebenen Sensorkapsel, wobei konkret die Kapsel 3 zu sehen ist. Durch Zugabe von DODAB vergrößert sich die Dicke der markierten Zwischenschicht (sensitive Schicht).

Die Fluoreszenzintensität von Kapseln Nr. 3 nach Zugabe von 0,05 M DODAB ist dagegen in Figur 8 dargestellt.

### Literatur

1. Battersby, Bronwyn Jean et. al. Patent WO 00/32542, Juni 2000
2. Payan, Donald US Patent 20010006787, A1, Juli 2001**,**
3. Still, et. al. US Patent 5,565,324, Oktober 1996**;** Still et.al., US Patent 6,001,579, März 1999
4. Norrman, Nils, Patent EP 1190256, März 2002
5. Trau, Mathias et.al. WO 99/24458, Mai 1999
6. Donath, E. et.al. WO 99/47252, März 1999
7. Spiro, A.; Lowe, M.; Brown, D. Appl. Env. Microbiology 66, 2000, 4258
8. Gaponik, N. Radtchenko, I.L.; Sukhorukov, G.B., Weller, H., Rogach, A.L. Adv. Mater. 14,2002,879
9. E. Donath, G.B. Sukhorukov, F. Caruso, S.A. Davis, H. Möhwald, Angew. Chem. Int. Ed. 1998, 37, 2002.
10. R. Steitz, V. Leiner, R. Siebrecht, R. v. Klitzing, Colloids a. Surf. A, 2000, 163, 63.
11. G. Ibarz, L. Dähne, E. Donath, H. Möhwald "Smart Micro- and Nanocontainers for Storage, Transport and Release" Adv. Mater. 13 (2001) 1324-1327.
12. L. Dähne, E. Donath, S. Leporatti, H. Möhwald, "Synthesis of micro reaction cages with defined chemical properties" J. Amer. Chem. Soc. 123 (2001), 5431-5436.
13. H. Härmä, "Particle technologies in diagnostics" TEKES Technology Review 126/2002

## Patentansprüche

1. Kapseln
- mit einem Durchmesser kleiner als 100 µm, und
- einer Hülle, die zumindest drei Polyelektrolytschichten aufweist, wobei die Hülle lediglich einen Farbstoff enthält und zumindest eine dieser drei Polyelektrolytschichten mit dem Farbstoff markiert ist, der mit einem sensitiven Material kovalent in hoher Konzentration verknüpft ist, wobei das sensitive Material auf veränderte Umgebungsbedingungen durch Volumenzunahme oder Volumenabnahme reagieren kann,
- wobei die Konzentration des Farbstoffs so hoch ist, daß der Farbstoff mit sich selbst Dimere, Aggregate oder Excimere ausbildet, die zu einem Selfquenching der Fluoreszenz oder zur Ausbildung einer neuen Emissionsbande führen, und
- wobei das sensitive Material ein organischer Polyelektrolyt ist.

2. Kapseln nach Anspruch 1, **dadurch gekennzeichnet, daß** es sich bei dem sensitiven Material um ein Material handelt, das bei Veränderung seiner Umgebungsbedingungen entweder quillt oder schrumpft und so sein Volumen ändert.

3. Kapseln nach Anspruch 2, **dadurch gekennzeichnet, daß** es sich bei den Umgebungsbedingungen um pH-Wert, Salzkonzentration, Temperatur, adsorbierte Komponenten, Enzyme, Konzentration eines Stoffes, physikalische Parameter, das Lösungsmittel beeinflussende oder mit dem sensitiven Material reagierende Komponenten sowie mischbare Lösungsmittelanteile handelt.

4. Kapseln nach einem der Ansprüche 1 bis 3, **dadurch gekennzeichnet, daß**
die Konzentration des Farbstoffs der Beziehung
Masse sensitiven Materials : Masse Farbstoff < 500 : 1
genügt.

5. Kapseln nach einem der Ansprüche 1 bis 4, **dadurch gekennzeichnet, daß** die mit Farbstoff markierte Schicht eine Dicke von 1 nm bis 1 um aufweist.

6. Kapseln nach einem der vorangehenden Ansprüche, **dadurch gekennzeichnet, daß** es sich bei dem Farbstoff um Fluoreszenzfarbstoffe oder emittierende Nanopartikel handelt.

7. Kapseln nach einem der vorangehenden Ansprüche, **dadurch gekennzeichnet, daß** die Kapseln hohl sind und sich in ihrem von der Hülle begrenzten Innenraum Makromoleküle befinden.

8. Kapseln nach einem der Ansprüche 1 bis 6, **dadurch gekennzeichnet, daß** die Kapseln einen modifizierten Kern aufweisen, der sensorische oder Koordinierungseigenschaften besitzt.

9. Kapseln nach einem der vorangehenden Ansprüche, **dadurch gekennzeichnet, daß** die Hülle für Moleküle bis zu einer gewissen Größe permeabel sind.

10. Kapseln nach einem der Ansprüche 1 bis 6, **dadurch gekennzeichnet, daß** die Kapseln einen festen Kern aufweisen, der von der Hülle umgeben ist.

11. Kapseln nach einem der vorangehenden Ansprüche, **dadurch gekennzeichnet, daß** die Kapseln einen mittleren Durchmesser von weniger als 10 µm, bevorzugt von weniger als 1 µm besitzen.

12. Kapseln nach einem der vorangehenden Ansprüche, **dadurch gekennzeichnet, daß** die Kapseln durch das Layer-by-Layer Verfahren hergestellt sind.

13. Kapseln nach einem der vorangehenden Ansprüche, **dadurch gekennzeichnet, daß** die Kapseln zur Markierung oder Codierung von technischen Erzeugnissen, Partikeln, Zellen, Gewebe, Organen oder Organismen biologischen Ursprungs dienen.

14. Verwendung der Kapseln nach einem der vorangehenden Ansprüche als Bibliothek von Reporter-Partikeln oder kodierten Farbpartikeln zur Identifikation von Substanzen und/ oder der Markierung von Prozessen.

15. Verwendung der Kapseln nach einem der vorangehenden Ansprüche in der medizinischen Diagnostik, der kombinatorischen Chemie, der Genomik und Proteomik, der Biologie und Biotechnologie und der Technik.

## Claims

1. Capsules
- having a diameter of less than 100 µm and
- a shell which has at least three polyelectrolyte layers, the shell containing only one dye and at least one of these three polyelectrolyte layers being marked with the dye which is covalently linked in high concentration to a sensitive material, the sensitive material being able to react to changed ambient conditions by a volume increase or volume decrease,
- the concentration of the dye being so high that the dye forms, with itself, dimers, aggregates or excimers which lead to self-quenching of the fluorescence or to the formation of a new emission band, and
- the sensitive material being an organic polyelectrolyte.

2. Capsules according to Claim 1, **characterized in that** the sensitive material is a material which either swells or shrinks and thus changes its volume in the case of a change in its ambient conditions.

3. Capsules according to Claim 2, **characterized in that** the ambient conditions are pH, salt concentration, temperature, adsorbed components, enzymes, concentration of a substance, physical parameters, components which influence the solvent or react with the sensitive material, and miscible solvent fractions.

4. Capsules according to any of Claims 1 to 3, **characterized in that** the concentration of the dye satisfies the relationship of mass of sensitive material:mass of dye < 500:1.

5. Capsules according to any of Claims 1 to 4, **characterized in that** the layer marked with dye has a thickness of 1 nm to 1 µm.

6. Capsules according to any of the preceding Claims, **characterized in that** the dye comprises fluorescent dyes or emitting nanoparticles.

7. Capsules according to any of the preceding Claims, **characterized in that** the capsules are hollow and macromolecules are present in its interior bounded by the shell.

8. Capsules according to any of Claims 1 to 6, **characterized in that** the capsules have a modified core which has sensory or coordinating properties.

9. Capsules according to any of the preceding Claims, **characterized in that** the shell is permeable to molecules up to a certain size.

10. Capsules according to any of Claims 1 to 6, **characterized in that** the capsules have a solid core which is surrounded by the shell.

11. Capsules according to any of the preceding Claims, **characterized in that** the capsules have a mean diameter of less than 10 µm, preferably of less than 1 µm.

12. Capsules according to any of the preceding Claims, **characterized in that** the capsules are produced by the layer-by-layer process.

13. Capsules according to any of the preceding Claims, **characterized in that** the capsules serve for marking or coding technical products, particles, cells, tissues, organs or organisms of biological origin.

14. Use of the capsules according to any of the preceding Claims as a library of reporter particles or coded coloured particles for the identification of substances and/or the marking of processes.

15. Use of the capsules according to any of the preceding Claims in medical diagnosis, combinatorial chemistry, genomics or proteomics, biology and biotechnology and technology.

## Revendications

1. Capsules
- présentant un diamètre inférieur à 100 µm, et
- comportant une enveloppe qui présente au moins trois couches de polyélectrolytes, où l'enveloppe contient simplement un colorant, et au moins une de ces trois couches de polyélectrolytes est marquée avec le colorant, qui est lié de façon covalente selon une concentration élevée avec un matériau sensible, le matériau sensible pouvant réagir à des modifications des conditions environnementales par une augmentation de volume ou une réduction de volume,
- dans lesquelles la concentration du colorant est élevée au point que le colorant forme avec lui-même des dimères, des agrégats ou des excimères, qui entraînent une auto-extinction de la fluorescence ou la formation d'une nouvelle bande d'émission, et
- dans lesquelles le matériau sensible est un polyélectrolyte organique.

2. Capsules selon la revendication 1, **caractérisées en ce qu'**il s'agit pour ce qui est du matériau sensible d'un matériau qui soit gonfle soit se contracte lors de la modification de ses conditions environnementales, et modifie ainsi son volume.

3. Capsules selon la revendication 2, **caractérisées en ce qu'**il s'agit concernant les conditions environnementales de la valeur du pH, de la concentration en sel, de la température, de composants adsorbés, d'enzymes, de la concentration d'une substance, de paramètres physiques, de composants influençant le solvant ou réagissant avec le matériau sensible, ainsi que de fractions de solvants miscibles.

4. Capsules selon l'une des revendications 1 à 3, **caractérisées en ce que** la concentration du colorant satisfait à la relation
masse du matériau sensible : masse du colorant < 500 :1.

5. Capsules selon l'une des revendications 1 à 4, **caractérisées en ce que** la couche marquée avec le colorant présente une épaisseur comprise entre 1 nm et 1 µm.

6. Capsules selon l'une des revendications précédentes, **caractérisées en ce qu'**il s'agit concernant le colorant de colorants de fluorescence ou de nanoparticules à émission.

7. Capsules selon l'une des revendications précédentes, **caractérisées en ce que** les capsules sont creuses et que des macromolécules se trouvent dans leur espace interne délimité par l'enveloppe.

8. Capsules selon l'une des revendications 1 à 6, **caractérisées en ce que** les capsules possèdent un noyau modifié qui possède des propriétés sensorielles ou de coordination.

9. Capsules selon l'une des revendications précédentes, **caractérisées en ce que** les enveloppes des molécules sont perméables jusqu'à une certaine taille.

10. Capsules selon l'une des revendications 1 à 6, **caractérisées en ce que** les capsules présentent un noyau solide qui est entouré de l'enveloppe.

11. Capsules selon l'une des revendications précédentes, **caractérisées en ce que** les capsules possèdent un diamètre moyen inférieur à 10 µm, de préférence inférieur à 1 µm.

12. Capsules selon l'une des revendications précédentes, **caractérisées en ce que** les capsules sont fabriquées selon le principe du dépôt couche par couche.

13. Capsules selon l'une des revendications précédentes, **caractérisées en ce que** les capsules servent au marquage ou au codage de produits techniques, de particules, de cellules, de tissus, d'organes ou d'organismes d'origine biologique.

14. Utilisation des capsules selon l'une des revendications précédentes en tant que bibliothèque de particules reporter ou de particules colorées codées destinées à l'identification de substances et/ou au marquage de processus.

15. Utilisation des capsules selon l'une des revendications précédentes dans le diagnostic médical, la chimie combinatoire, la génomique et la protéomique, la biologie et la biotechnologie, ainsi que la technique.
